(19) Europäisches Patentamt
European Patent Office
Office européen des brevets

(11) Publication number: 0 190 457
A1

(12) **EUROPEAN PATENT APPLICATION**

(21) Application number: 85116452.5

(22) Date of filing: 21.12.85

(51) Int. Cl.[4]: C 07 D 487/04
C 07 D 231/38, C 07 D 231/40
//A61K31/415, (C07D487/04, 235:00, 231:00)

(30) Priority: 07.02.85 IT 1941885

(43) Date of publication of application: 13.08.86 Bulletin 86/33

(84) Designated Contracting States: AT BE CH DE FR GB IT LI LU NL SE

(71) Applicant: CAMILLO CORVI S.p.A.
via S. Marco 18
Milan(IT)

(72) Inventor: Vicentini, Chiara Beatrice
via Matteotti 22/C
I-45031 Arquà Polesine Rovigo(IT)

(72) Inventor: Veronese, Augusto
via Ercole Iò D'Este, 54
I-44100 Ferrara(IT)

(72) Inventor: Guarneri, Mario
Via Pomposa 42
I-44100 Ferrara(IT)

(74) Representative: Klausner, Erich et al,
c/o Ufficio Internazionale Brevetti Ing. C. Gregorj S.p.A.
Via Dogana 1
I-20100 Milan(IT)

(54) **Derivatives of 3-methyl-imidazo ]4,5-c[pyrazole having therapeutic activity and a process for the preparation theref.**

(57) The present invention concerns novel derivatives of 3-methyl-imidazo [4,5-c] pyrazole of formula

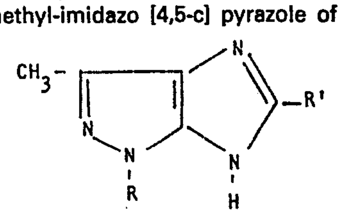

(I)

Said compounds have therapeutical activity, in particular said compounds are endowed with an intense CNS depressant activity. The invention furthermore concerns a process for preparing the compounds of formula (I).

EP 0 190 457 A1

DESCRIPTION

The present invention relates to derivatives of 3-methyl-imidazo [4,5 -c] pyrazole having therapeutical activity. Furthermore, this invention relates also to a process for the preparation of said derivatives.

More particularly, the derivatives of this invention corres pond to the following structural formula

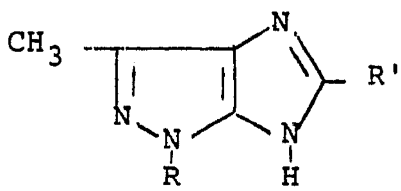

wherein R is a $(C_1-C_4)$ alkyl group, preferably a methyl group, or a 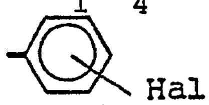 group in which Hal represents a halogen, preferably Cl or F, and R' is a $(C_1-C_4)$ alkyl group, preferably a methyl group, or a phenyl group.

The compounds of formula (I) of the present invention are endowed with an intense depressant activity on the central nervous system, in particular they exhibit the following activities : anticonvulsant, sedative, analgesic and hypothermizing. These activities occur at doses a lower than the toxic ones. In particular, the acute toxicity $LD_{50}$ per os in the mouse is of 150 mg/kg.

Few bibliographic references to the nucleus of imidazo[4,5-c] pyrazole are found : up to a few years ago, the only two known representative compounds

were 1-phenyl-5-hydroxy-imidazo [4,5-c] pyrazole (v.A.Dornow, E.Hinz,Chem.Ber.91,1834(1958) and 1-phenyl-3-methyl-5- mercaptomidazole [4,5-c] pyrazole (v.I. Grandberg, G.V. Klyuchko, Zh. Obshch. Khim. 32, 1898 (1962).

More recently, two research groups attain the synthesis of other members, the pharmacology of which had not, however, been investigated at all. (v.M. Lange,R.Quell, H. Lettau, H.Schubert,Z.Chem. 17,94 (1977);V.Sudarsanam, K.Nagarajan, K.Rama Rao, S.J.Shenoy, Tetrahedron Letters 21, 4757 (1980); K.Nagarajan, V.Sudarsanam,S.J. Shenoy, K.Rama Rao, Indian J. Chem. Sect. B 21B, 997 (1982).

The above mentioned members were obtained a) by Curtius transposition and cyclization of 4-carbazolyl-5-aminopyrazoles, b) by cyclization of 4-nitro-5-benzylaminopyrazoles, c) by reaction of 4,5-diaminopyrazoles with carbon sulphide and d) by cycloaddition of diazomethane on the double bond C=C of 5-nitroimidazoles.

It has now been found that acylation of 5-aminopyrazoles of formula (II) (v.Scheme A) with benzoyl chloride or acetic anhydride leads to the corresponding 5-acylaminopyrazoles of formula (III) which are reduced to 5-alkylaminopyrazoles of formula (IV) by means of $LiAlH_4$ in tetrahydrofuran. Nitrosation with amyl nitrite and hydrochloric acid leads to the 4-nitroso-5-alkylaminopyrazoles of formula (V), which

are cyclized to imidazo ⌈4,5-c⌋ pyrazoles of formula (I), by refluxing in pyridine.

According to the process of this ivention, the above mentioned compounds are obtained in good yields as crystalline products, except the 5-alkylaminopyrazoles of formula (IV) which generally occur as oils. In certain cases, the compounds of formula (I) crystallize with water molecules, however, the non-hydrated product can easily be obtained by heating at reduced pressure, over $P_2O_5$, over a period of 48 hours.

The spectral (IR and $^1$H-NMR) data of the compounds of formula (I) are consistent with the structures as reported.

SCHEME A

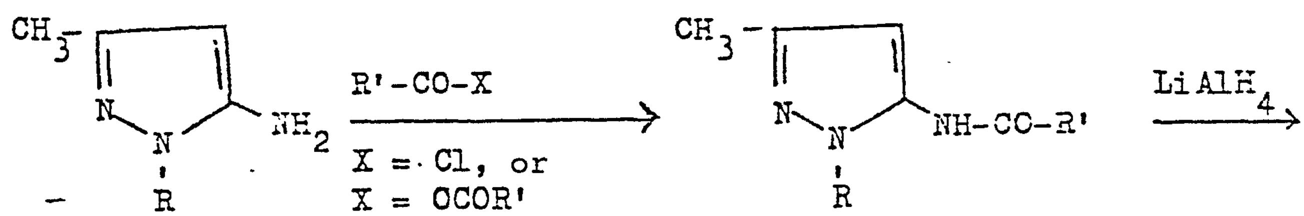


(II a-r) (III a-r)

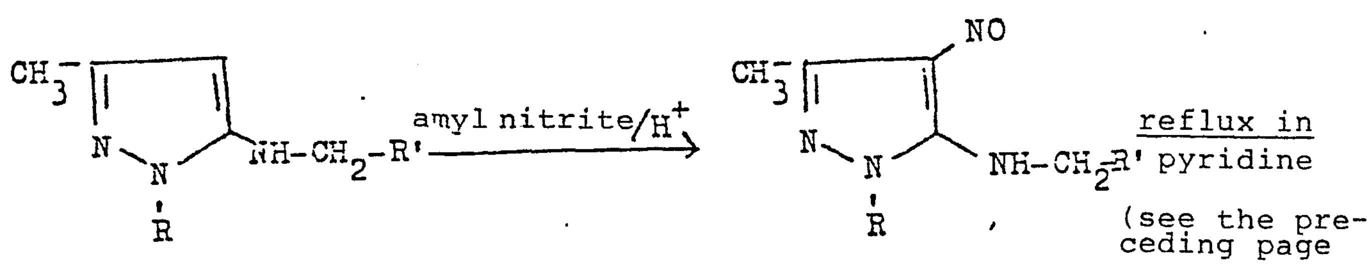


(IV a-r) (V a-r)

reflux
in pyridine

(see the preceeding page)

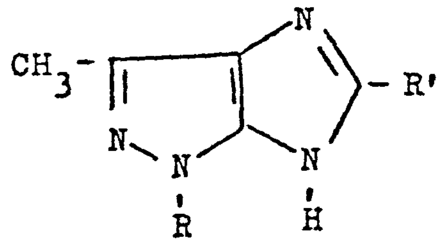

(I a-r)

| (I)-(V) | a | b | c | d | e | f | g | h | i |
|---|---|---|---|---|---|---|---|---|---|
| R | $CH_3$ | $CH_3$ | $C_6H_5$ | $C_6H_5$ | p.$ClC_6H_4$ | p.$ClC_6H_4$ | m.$ClC_6H_4$ | m.$ClC_6H_4$ | o.ClC$_6$H |
| R' | $CH_3$ | $C_6H_5$ | $CH_3$ | $C_6H_5$ | $CH_3$ | $C_6H_5$ | $CH_3$ | $C_6H_5$ | $CH_3$ |

| (I)-(V) | l | m | n | o | p | q | r |
|---|---|---|---|---|---|---|---|
| R | o.$ClC_6H_4$ | p.$FC_6H_4$ | p.$FC_6H_4$ | m.$FC_6H_4$ | m.$FC_6H_4$ | o.$FC_6H_4$ | o.$FC_6H_4$ |
| R' | $C_6H_5$ | $CH_3$ | $C_6H_5$ | $CH_3$ | $C_6H_5$ | $CH_3$ | $C_6H_5$ |

Example 1

1,3-dimethyl-5-phenylimidazo [4,5-] pyrazole (I b)

A suspension of 1,3-dimethyl-5-aminopyrazole (IIb) (4.445g, 0.04 mole) and benzoyl chloride (0.23ml, 0.02 mole) in anhydrous benzene is refluxed for one hour and then filtered, upon evaporation of the solvent, a solid is obtained, corresponding to 1,3-dimethyl-5-benzamidopyrazole (III b), which is purified by

crystallization from ethyl acetate/petroleum ether (3,26g, yield 76%, m.p. 135°-136°C).

3.26g of 1,3-dimethyl-5-benzamidopyrazole (III b) (0.015 mole), dissolved into anhydrous tetrahydrofuran (19ml), are added to a suspension of $LiAlH_4$ (1.13g,0.030 mole) in anhydrous tetrahydrofuran (11 ml) under a nitrogen stream. The suspension is refluxed for 4 hours. Then, water (1.3 ml),a 15% sodium hydroxide water solution (1.3ml) and water (4 ml) are added. The precipitate which separates is filtered off, the solution is evaporated and the residue is taken up with ethyl acetate. The organic solution, after having been washed with water, is anhydrified over anhydrous sodium sulphate and desiccated. 1,3-Dimethyl-5-benzylaminopyrazole (IV b) is obtained (2.16g, yield 71%,m.p. 94°-95°C).

Two drops of concentrated hydrochloric acid and subsequently 1.44 ml (0.0107 mole) of amyl nitrite are added dropwise to a solution, cooled to 0°C, of (IV b) (2.16g, 0.0107 mole) in ethanol (10ml). After maintaining the solution for one hour at room temperature, it is evaporated to dryness; the so obtained solid is taken up with ether and filtered off. 1.78g of 1,3-dimethyl-4-nitroso-5-benzylaminopyrazole (V b) (yield 72%, m.p. 140-142 C) are obtained.

The product (V b) so obtained (1.78g, 0.0077 mole) is refluxed in pyridine 5 minutes. The reaction is monitored by thin layer chromatography (F254 Merck silicon gel ), using

ethyl acetate as element until the disappearance of product (V b). After evaporating the solvent, the residue is washed with a little ethyl ether and is filtered off. The product which formsis 1,3-dimethyl-5-phenylimidazo [4,5-c] pyrazole of formula (Ib): 127g, yield 71%; m.p. 195-196°; elemental analysis for $C_{12}H_{12}N_4 \cdot H_2O$ (M.W.= 230.28) calc.% C 62.59, H 6.13, N 24.33; found % C 62.59, H 6.04, N 24.26; I.R. (KBr) $\nu$ $cm^{-1}$ 3050 v.br., 1560; $^1$H-NMR ($CDCl_3$-$d_6$DMSO 1:1) 2.35 (3H), 3.8 (3H), 7.4-8.2 (m5H), 12.3 (br,1 H).

Example 2

1-Phenyl-3-methyl-5-methylimidazo [4,5-c] pyrazole (I c)

1-Phenyl-3-methyl-5-acetamidopyrazole (III c) is prepared from 1-phenyl-3-methyl-5-aminopyrazole (II c) by treatment with acetic anhydride at 100°C, as described by A. Michaelis 339, 141.

The 1-phenyl-3-methyl-5-acetamidopyrazole (III c) (5.59g, 0.026 mole), dissolved into anhydrous tetrahydrofuran (33 ml), is added to a suspension of $LiAlH_4$ (1.97g, 0.052 mole) in anhydrous tetrahydrofuran (20ml) under a nitrogen stream. The suspension is refluxed for 4 hours. Then, water (2.3 ml) , a 15% sodium hydroxide water solution (2.3 ml) and water (7ml) are added. The precipitate which separates is filtered off the solution is evaporated and the residue is taken up with ethyl acetate. The organic solution, after

having been washed with water, is anhydrified over anhydrous sodium sulphate and desiccated. 4.44 g (yield 85%) of 1-phenyl-3-methyl-5-ethylaminopyrazole (IV e) are obtained as an oil.

Five drops of concentrated hydrochloric acid and subsequent 2.95 ml (0.022 mole ) of amyl nitrite are added dropwise to a solution cooled to 0°C of (IV c) (4.44g, 0.022 mole) in ethanol (22 ml). After maintaining the solution at room temperature for one hour, it is evaporated to dryness : the solid as obtained is washed with water and filtered off 3.55g of 1-phenyl-3-methyl-4-nitroso-5-ethylaminopyrazole (V c) (yield 70%, m.p. 112-114 C) are obtained.

The so obtained product (V c) (3.55g, 0.015 mole) is refluxed in pyridine for 10 minutes.
The reaction is monitored by thin layer chromatography (F.254 Merck silicon gel plates) using as element a 1:1 mixture of ethyl acetate/petroleum ether in order to monitor the disappearance of the product (V c). After evaporating the solvent, the crude product is purified over a silica gel column, by eluting with a 4:1 mixture of ethyl acetate/petroleum ether. The fractions corresponding to the desired product are evaporated to dryness, to give a crystalline product which is washed with little ethyl ether and filtered off. The product so obtained

is 1-phenyl-3-methyl-5-methylimidazo [4,5-c] pyrazole of formula (Ic) : 2.68 g. yield 82%; m.p. 217°C: elemental analysis for $C_{12}H_{12}N_4$ (M.W. = 212.26) calc.% 67.90, H 5.70, N 26.39; found % C 68.01, H 5.47, N 26.21;I.R. (KBr) $cm^{-1}$ 3050 v.br., 1550; $^1$H-NMR ($CDCl_3$-$d_6$CMSO 1:1) 2.4 (3H), 2.5 (3H), 7.8-8.1 (m,5H), 11.9 (br.1H).

In the same way, were prepared the imidazo [4,5-c] pyrazoles (Ia), (Ie),(Ig), (Ii), (Im), (Io), (Iq), starting from 1,3-dimethyl-5-aminopyrazole, 1-p-chloro-phenyl-3-methyl-5-aminopyrazole, 1-m-chlorophenyl-3-methyl-5-aminopyrazole, 1-o-chlorophenyl-3-methyl-5-aminopyrazole, 1-p-fluorophenyl-3-methyl-5-aminopyrazole, 1-m-fluorophenyl-3-methyl-5-aminopyrazole, and 1-o-flurophenyl-3-methyl-5-aminopyrazole, respectively.

Example 3

1-Phenyl-3-methyl-5-phenylimidazo [4,5-c] pyrazole (Id)

2.3. ml of benzoyl chloride (0.02 mole) dissolved into 50 ml of ethyl acetate are added dropwise to a mixture, kept under vigorous stirring,of 1-phenyl-3-methyl-5-aminopyrazole (II d) (3.46g, 0.02 mole), dissolved into 90 ml of ethyl acetate and 0.5 N sodium bicarbonate (44ml). After stirring the mixture for 4 hours at 60°C, the organic phase is washed with 5%

sodium carbonate, water, 1N hydrochloric acid and water. The organic phase is then anhydrified over anhydrous sodium sulphate and desiccated. A solid is obtained, corresponding to 1-phenyl-3-methyl-5-benzamidopyrazole (III d), which is purified by crystallization from ethyl acetate:petroleum ether (3.77, yield 68%, m.p. 119-121°C). The 1-phenyl-3-methyl-5-benzamidopyrazole (III d) (3.77g, 0.0136 mole), dissolved into anhydrous tetrahydrofuran (17ml) , is added to a suspension of $LiAlH_4$ (1.03g, 0.027 mole), in anhydrous tetrahydrofuran (11ml) under a nitrogen stream. The suspension in refluxed during 4 hours. Then are added; water (1.2 ml), a 15% sodium hydroxide aqueous solution (1.2 ml) and water (3.6 ml). The precipitate which separates is filtered off, the solution is evaporated and the residue is taken up with ethyl acetate. The organic solution, after having been washed with water, is anhydrified over anhydrous sodium sulphate and desiccated. 1-Phenyl-3-methyl-5-benzylaminopyrazole (IV d), an oil, 3.19 g, (yield 89%) is obtained.

Three drops of concentrated hydrochloric acid and subsequently 1.61 ml (0.012 mole) of amyl nitrite are added dropwise to a solution, cooled to 0°C, of (IV d) (3.19 g, 0.012 mole) in ethanol (11 ml). After maintaining the solution one hour at room temperature, the solid which separates

spontaneously from the reaction mixture is filtered off. 1.92g of 1-phenyl-3-methyl-4-nitroso-5-benzylaminopyrazole (V d) (yield 54%, m.p. 119°C) are obtained.

The product so obtained (V d) (1.92 g, 0.0065 mole) is refluxed in pyridine for 15 minutes. The reaction is monitored by thin layer chromatography (F254 Merck silica gel plates) using 1:1 ethyl acetate/petroleum ether as eluent and monitoring the disappearance of the product (V d). After evaporating the solvent, the crude product is purified over a silica gel column, by eluting with a 3:7 mixture of ethyl acetate/petroleum ether. The fractions corresponding to the desired product are desiccated, to give a crystalline product which is washed with a little ethyl ether and filtered off. The so obtained product is 1-phenyl-3-methyl-5-phenylimidazo [4,5-c] pyrazole of formula (I d): 1.19 g, yield 62% m.p. 273°C; elemental analysis for $C_{17}H_{14}N_4 \cdot H_2O$ (M.W. = 292.35) calc. % C 69.84, H 5.52, N 19.17; found % C 69.60, H 5.60 N 18.96; I.R. (KBr) 3050 v.br., 1550; $^1H$-NMR ($CDCl_3$-$d_6$DMSO 1:1) 2.5 (3H), 7.4-8.2 (m,10H), 12.3 (br, 1H).

In the same way were prepared the imidazo [4,5-c] pyrazoles (I f), (I h), (I l), (I n), (I p) (I r), starting from, respectively 1-p-chlorophenyl-3-methyl-5-aminopyrazole, 1-m-chlorophenyl-3-methyl-5-aminopyrazole, 1-o-chlorophenyl-3-

methyl-5-aminopyrazole, 1-p-fluorophenyl-3-methyl-5-aminopyrazole, 1-m-fluorophenyl-3-methyl-5-aminopyrazole, and 1-o-fluorophenyl-3-methyl-5-aminopyrazole.

Table 1. Imidazo [4,5-c] pyrazoles Ia-r

| Compound | | m.p. [°C] | formula (PM) |
|---|---|---|---|
| 1.3-dimethyl-5-methylimidazo [4,5-c] pyrazole | Ia | 90 | $C_7H_{10}N_4 \cdot \frac{1}{2} H_2O$ (159.19) |
| 1.3-dimethyl-5-phenylimidazo [4,5-c] pyrazole | Ib | 195-196 | $C_{12}H_{12}N_4 \cdot H_2O$ (230.28) |
| 1-phenyl-3-methyl-5-methylimidazo [4,5-c] pyrazole | Ic | 217 | $C_{12}H_{12}N_4$ (212.26) |
| 1-phenyl-3-methyl-5-phenylimidazo [4,5-c] pyrazole | Id | 273 | $C_{17}H_{14}N_4 \cdot H_2O$ (292.35) |
| 1-p.chlorophenyl-3-methyl-5-methylimidazo [4,5-c]pyrazole | Ie | 250-251 | $C_{12}H_{11}N_4Cl$ (246.7) |
| 1-p.chlorophenyl-3-methyl-5-phenylimidazo [4,5-c]pyrazole | If | 214 | $C_{17}H_{13}N_4Cl.H_2O$ (326.79) |
| 1-m.chlorophenyl-3-methyl-5-methylimidazo [4,5-c]pyrazole | Ig | 189-190 | $C_{12}H_{11}N_4Cl$ (246.7) |
| 1-m.chlorophenyl-3-methyl-5-phenylimidazo [4,5-c]pyrazole | Ih | 256-258 | $C_{17}H_{13}N_4Cl.H_2O$ (326.79) |
| 1-o.chlorophenyl-3-methyl-5-methylimidazo [4,5-c]pyrazole | Ii | 175-176 | $C_{12}H_{11}N_4Cl$ (246.7) |

Table 1 Imidazo /4,5-c/ pyrazoles Ia-r

| | Compound | | m.p./°C/ | formula (PM |
|---|---|---|---|---|
| 10) | 1-o.chlorophenyl-3-methyl-5-phenylimidazo /4,5-c/ pyrazole | Il | 174-175 | $C_{17}H_{13}N_4Cl$ (308.77) |
| .1) | 1-p.fluorophenyl-3-methyl-5-methylimidazo /4,5-c/ pyrazole | Im | 248-249 | $C_{12}H_{11}F_4$ (230.25) |
| .2) | 1-p.fluorophenyl-3-methyl-5-phenylimidazo /4,5-c/ pyrazole | In | 194-195 | $C_{17}H_{13}N_4F \cdot H_2O$ (310.34) |
| .3) | 1-m.fluorophenyl-3-methyl-5-methylimidazo /4,5-c/ pyrazole | Io | 188-189 | $C_{12}H_{11}N_4F \cdot H_2O$ (248.26) |
| .4) | 1-m.fluorophenyl-3-methyl-5-phenylimidazo /4,5-c/ pyrazole | Ip | 276-277 | $C_{17}H_{13}N_4F \cdot \frac{1}{2}H_2O$ (301.33) |
| .5) | 1-o.fluorophenyl-3-methyl-5-methylimidazo /4,5-c/ pyrazole | Iq | 194 | $C_{12}H_{11}N_4F$ (230.25) |
| .6) | 1-o.fluorophenyl-3-methyl-5-phenylimidazo /4,5-c/ pyrazole | Ir | 204-206 | $C_{17}H_{13}N_4F$ (292.32) |

| | | Elemental analysis %C | %H | %N | %C | %F | I.R. (KBr)* ν /cm$^{-1}$/ | $^1$H-NMR,** δ /ppm/ |
|---|---|---|---|---|---|---|---|---|
| ) | found | 53.01 | 6.96 | 34.98 | | | 3100 v.br.1560 | 2.3(3H),2.42(3H),3.8(3H),11.5(br,1H) |
| | calc. | 52.81 | 6.97 | 35.20 | | | | |
| ) | found | 62.59 | 6.04 | 24.26 | | | 3050 v.br.1560 | 2.35(3H),3.8(3H),7.4-8.2(m.5H),12.3(br.1H) |
| | calc. | 62.59 | 6.13 | 24.33 | | | | |
| ) | found | 68.01 | 5.47 | 26.21 | | | 3050 v.br.1550 | 2.4(3H),2.5(3H),7.8-8.1(m.5H),11.9(br.1H) |
| | calc. | 67.90 | 5.70 | 26.39 | | | | |
| ) | found | 69.60 | 5.60 | 18.96 | | | 3050 v.br.1550 | 2.5(3H),7.4-8.2(m,10H),12.3 (br,1H) |
| | calc. | 69.84 | 5.52 | 19.17 | | | | |
| ) | found | 58.53 | 4.49 | 22.68 | 14.50 | | 3000 v.br.1555 | 2.4(3H),2.5(3H),7.45-8.1($A_2B_2$,J=9Hz,4H), 12.0(br,1H) |
| | calc. | 58.42 | 4.49 | 22.71 | 14.37 | | | |
| ) | found | 62.62 | 4.49 | 17.26 | 10.71 | | 3080 v.br.,1545 | 2.5(3H),7.3-8.2(m,9H),12.2(br.1H) |
| | calc. | 62.48 | 4.63 | 17.15 | 10.85 | | | |
| ) | found | 58.51 | 4.61 | 22.84 | 14.16 | | 3100 v.br.,1550 | 2.4(3H),2.5(3H),7.1-8.1(m.4H),12.1(br,1H) |
| | calc. | 58.42 | 4.49 | 22.71 | 14.37 | | | |
| ) | found | 62.60 | 4.75 | 17.39 | 11.05 | | 3080 v.br.,1550 | 2.45(3H),7.2-8.2(m,9H),12.8 (br,1H) |
| | calc. | 62.48 | 4.63 | 17.15 | 10.85 | | | |
| ) | found | 58.23 | 4.41 | 23.01 | 14.58 | | 3050 v.br.,1550 | 2.35(3H),2.40(3H),7.3-7.7(m,4H),12.0 (br,1H) |
| | calc. | 58.42 | 4.49 | 22.71 | 14.37 | | | |
| 0) | found | 65.99 | 4.29 | 18.30 | 11.73 | | 3080 v.br.,1550 | 2.45(3H),7.35-8.2(m,9H),12.7(br,1H) |
| | calc. | 66.13 | 4.24 | 18.15 | 11.48 | | | |
| 1) | found | 62.43 | 4.83 | 24.53 | | 8.16 | 2950 v.br.,1555 | 2.4(3H),2.5(3H),7.2-8.2(m,4H),12.1(br,1H; |
| | calc. | 62.60 | 4.82 | 24.33 | | 8.25 | | |

| | | %C | %H | %N | %C | %F | I.R. (KBr)* ν /cm$^{-1}$/ | $^1$H-NMR** δ/ppm/ |
|---|---|---|---|---|---|---|---|---|
| 12) | found | 65.79 | 4.84 | 17.86 | | 5.99 | 3050v.br.,1550 | 2.45(3H),7.25-8.3(m.9H),12.8 (br,1H) |
| | calc. | 65.80 | 4.87 | 18.05 | | 6.12 | | |
| 13) | found | 58.33 | 5.20 | 22.80 | | 7.62 | 3080 V.br.,1550 | 2.35(3H), 2.45(3H),6.9-8.0(m,4H),12.2(1H) |
| | calc. | 58.06 | 5.28 | 22.57 | | 7.65 | | |
| 14) | found | 67.96 | 4.68 | 18.50 | | 6.19 | 3080 v.br.,1560 | 2.45(3H),7.0-8.3(m,9H), 12.8(br,1H) |
| | calc. | 67.76 | 4.68 | 18.59 | | 6.31 | | |
| 15) | found | 62.39 | 4.84 | 24.58 | | 8.06 | 3050 v.br.,1550 | 2.35(3H),2.40(3H),7.3-7.9 (m,4H),12.0(br.1H) |
| | calc. | 62.60 | 4.82 | 24.33 | | 8.25 | | |
| 16) | found | 69.89 | 4.54 | 19.18 | | 6.42 | 3080 v.br.,1560 | 2.45(3H),7.3-8.2(m,9H),12.7(br,1H) |
| | calc. | 69.85 | 4.48 | 19.17 | | 6.50 | | |

* The I.R. spectra were determined by a Perkin Elmer 1756 spectrophotometer

** The $^1$H-NMR spectra were determined at 90 MHz, with tetramethylsilane as an internal standard by employing a Perkin Elmer R32 spectrophotometer : the signals are singlets; on the contrary the meaning is specified. The spectra of the Ia-f compounds were determined in 1:1 $CDCl_3$-$d_6$DMSO, while the spectra of the Ig-r compounds were determined in $d_6$DMSO.

CLAIMS

1. Derivatives of 3-methyl-imidazo [4,5-c] pyrazole, having an intense depressant activity on the central nervous system, of the formula

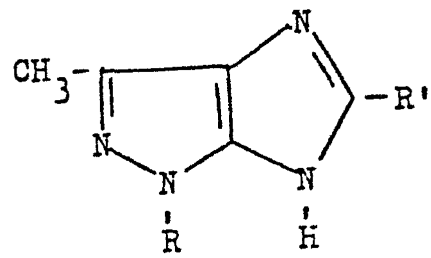

(I)

in which R is a $(C_1-C_4)$-alkyl group , or a 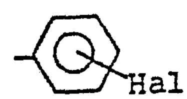 group in which Hal represents a halogen, and R' is a $(C_1-C_4)$-alkyl group, or a phenyl group.

2. A process for preparing the derivatives of 3-methyl-imidazo [4,5-c] pyrazole according to Claim 1, characterized in that a 5-acylaminopyrazole of formula (III) is prepared by treating with benzoyl chloride or acetic anhydride a 5-aminopyrazole of formula (II), then the above 5-acylaminopyrazole of formula (III) is reduced by lithium-aluminum hydride in tetrahydrofuran, to give the corresponding 5-alkylaminopyrazole of formula (IV) which, by treatment with amyl nitrite and hydrochloric acid, gives the corresponding 4-nitroso- derivative of formula (V) which, by reflux heating in pyridine, cyclizes, to give the corresponding 3-methylimidazo [4,5-c] pyrazole of formula (I).

CLAIMS FOR AUSTRIA

1. A process for preparing derivatives of 3-methylimidazo [4,5-c] pyrazole, having an intense depressant activity on the central nervous system , of the formula :

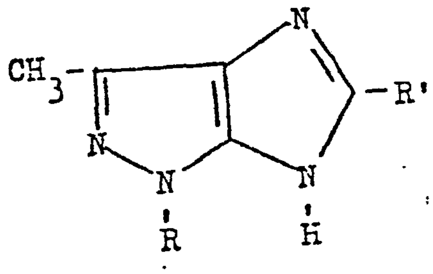

(I)

in which R is a $(C_1-C_4)$-alkyl group, or a 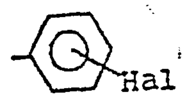 group in which Hal represents a halogen, and R' is a $(C_1-C_4)$ - alkyl group, or a phenyl group, characterized in that a 5-acylaminopyrazole of formula (III) is prepared by treating with benzoyl chloride or acetic anhydride a 5-aminopyrazole of formula (II), then the above 5-acylaminopyrazole of formula (III) is reduced by lithium-aluminum hydride in tetrahydrofuran, to give the corresponding 5-alkylaminopyrazole of formula (IV) which, by treatment with amyl nitrite and hydrochloric acid, gives the corresponding 4-nitroso-derivative of formula (V) which, by reflux heating in pyridine, cyclizes, to give the corresponding 3-methylimidazo [4,5-c] pyrazole of formula (I).

European Patent Office

# EUROPEAN SEARCH REPORT

0190457
Application number

EP 85116452.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | ZEITSCHRIFT FÜR CHEMIE, vol. 17, 1977<br>M. LANGE et al. "Heterobicyclen; Imidazo[4,5-c] pyrazole aus 4-Nitro-5-benzylaminopyrazolen"<br>pages 94-95<br>* Page 94 *<br>-- | 1,2 | C 07 D 487/04<br>C 07 D 231/38<br>C 07 D 231/40/<br>A 61 K 31/415<br>(C 07 D 487/04<br>C 07 D 235:00<br>C 07 D 231:00) |
| A | ORGANIC PREPARATIONS AND PROCEDURES INTERNATIONAL, vol. 16, no. 1, February 1984<br>M.R.H. ELMOGHAYAR et al. "Reactions with heterocyclic amidines. Synthesis of some new azolylthiourea derivatives"<br>pages 1-10<br>* Page 5 *<br>---- | 1,2 | |
| | | | **TECHNICAL FIELDS SEARCHED (Int. Cl.4)** |
| | | | C 07 D 487/00<br>C 07 D 231/00 |

The present search report has been drawn up for all claims

| Place of search | Date of completion of the search | Examiner |
|---|---|---|
| VIENNA | 21-04-1986 | PETROUSEK |

**CATEGORY OF CITED DOCUMENTS**

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document